# EUROPÄISCHE PATENTANMELDUNG

(11) **EP 3 623 005 A1**
(43) Veröffentlichungstag der Anmeldung: **18.03.2020**
(21) Anmeldenummer: 18194056.0
(22) Anmeldetag: 12.09.2018
(51) Int. Cl.: A61N 1/375, F16J 15/3272, A61N 1/362, H01R 13/504, A61N 1/36, F16J 15/16

(54) **DICHTUNGSRING AUS POLYSULFON UND FLÜSSIG-SILIKON**

(71) Anmelder: BIOTRONIK SE & Co. KG, 12359 Berlin (DE)
(72) Erfinder: Hartmann-Bax, Kathy, 14947 Nuthe-Urstromtal (DE); Lehmann, Stefan, 10245 Berlin (DE)
(74) Vertreter: Keck, Hans-Georg

(57) **Zusammenfassung**

Die vorliegende Erfindung betrifft einen Dichtungsring für einen Header eines implantierbaren Geräts, aufweisend einen Außenring, welcher Polysulfon umfasst oder im Wesentlichen daraus besteht, und einen Innenring, welches ein Flüssig-Silikon umfasst oder im Wesentlichen daraus besteht, wobei der Innenring und der Außenring zueinander formschlüssig angeordnet sind. Die Erfindung betrifft weiterhin ein Verfahren zur Herstellung eines solchen Dichtungsrings sowie eine Kontaktbuchse und ein implantierbares Gerät aufweisend einen solchen Dichtungsring.

## Beschreibung

Die vorliegende Erfindung betrifft einen Dichtungsring, insbesondere zur Verwendung in einem Verbindungskopf (Header) eines implantierbaren Geräts, sowie ein Verfahren zu dessen Herstellung.

Isolierende Dichtelemente in steckbaren Kontaktbuchsen in Verbindungsköpfen bzw. Headern von implantierbaren Geräten sind im Stand der Technik bekannt. Der Header dient hierbei insbesondere dazu, elektrische Impulse von Kern des implantierbaren Geräts zu einer in den Header gesteckten Elektrodenleitung zu leiten, ggf. auch in die umgekehrte Richtung. Die Bestandteile des Headers, insbesondere die Kontaktbuchse, werden typischerweise in einen Block aus Epoxidharz gegossen, um diese vor den Einflüssen der Zielumgebung zu schützen. Die eingangs genannten Dichtelemente sollen insbesondere eine Potentialtrennung zwischen den elektrisch leitenden Kontakten im Header gewährleisten. Weiterhin können solche Dichtungselemente als Anbindungsfläche für das oben genannte Epoxidharz sowie zur Herstellung eines bestimmten Abstandes zwischen den elektrisch leitenden Kontakten dienen.

Beispielsweise offenbart US 2009/0177167 A1 isolierende Dichtelemente in steckbaren Kontaktbuchse, welche aus einem Kern bestehen, der zur Wahrung der Distanz zwischen zwei elektrischen Kontakten, als Anschlag für die Montage, als Anbindungsfläche für Fügestoffe und der mechanischen Fixierung einer Dichtung ("wiper seal") dient. Die offenbarten Materialien für diesen Kern sind Polykristalline, einfache Kristalline oder aber kristallines Saphir.

Auch US 2016/0184594 A1 beschreibt die Verwendung beider Materialien im Header.

Ungeachtet dessen besteht nach wie vor ein Bedarf an geeigneten Designs für solche Dichtelemente, welche die oben genannten Aufgaben so gut wie möglich erfüllen.

Der generelle Aufbau von steckbaren Kontaktbuchsen in medizinischen Implantaten ist beispielsweise in den Dokumenten US 2015/0018909 A1, US 8,751,002 B2 oder US 2009/0017668 A1 offenbart.

Weiterhin beschreibt das Dokument US 2011/0059639 A1 die Verwendung von Silikon zur Dichtung und zur elektrischen Isolation in Bezug auf Kontaktbuchsen für medizinische Implantate. US 2009/0017668 A1 beschreibt den Gebrauch von *liquid silicone rubber* (LSR) für isolierende Dichtelemente in steckbaren Kontaktbuchsen.

Basierend auf diesem Hintergrund ist es die Aufgabe der vorliegenden Erfindung, verbesserte Dichtelemente sowie Verfahren zur deren Herstellung zur Verfügung zu stellen.

Diese Aufgabe wird durch einen Dichtungsring mit den Merkmalen des Anspruchs 1, einem Verbindungskopf mit den Merkmalen des Anspruchs 7, einem implantierbaren Gerät mit den Merkmalen des Anspruchs 8 und ein Verfahren zur Herstellung eines Dichtungsringes mit den Merkmalen des Anspruchs 9 gelöst. Vorteilhafte Ausgestaltungen sind in den entsprechenden abhängigen Ansprüchen und der folgenden Beschreibung dargestellt.

Gemäß Anspruch 1 wird ein Dichtungsring zur Verwendung in einem Header eines implantierbaren Geräts zur Verfügung gestellt. Der erfindungsgemäße Dichtungsring weist hierbei die folgenden Komponenten auf:
- einen Außenring bzw. Kern, welcher Polysulfon umfasst oder im Wesentlichen daraus besteht, und
- einen Innenring, welcher ein Flüssig-Silikon umfasst oder im Wesentlichen daraus besteht,
wobei der Innenring und der Außenring zueinander formschlüssig angeordnet sind.

Als Polysulfon im Sinne der vorliegenden Erfindung wird insbesondere ein thermoplastischer Kunststoff mit einer Aryl-SO₂-Aryl Untereinheit bezeichnet. Nicht beschränkende Beispiele für Polysulfone umfassen Polysulfon CAS Nr. 25135-51-7), Polyethersulfon (CAS Nr. 25608-63-3) und Polyphenylensulfon (CAS Nr. 25608-64-4).

Als Flüssig-Silikon oder Flüssigsilikonkautschuk (*liquid silicone rubber*) im Sinne der vorliegenden Erfindung wird insbesondere ein thermoplastische Elastomer bezeichnet, welches neben Poly(organo)siloxanen, vernetzbare Gruppen wie etwa Vinylgruppen und Si-H-Gruppen umfasst, die typischerweise in einer Edelmetall-katalysierten Reaktion addiert werden. Daneben kann das Flüssig-Silikon Additive wie verstärkende Stoffe und/oder Füllstoffe beinhalten.

In einigen weiteren Ausführungsformen des erfindungsgemäßen Dichtungsrings ist vorgesehen, dass der Außenring spritzgegossen wird.

In einigen weiteren Ausführungsformen des erfindungsgemäßen Dichtungsrings ist vorgesehen, dass der Innenring spritzgegossen wird.

In einigen weiteren Ausführungsformen des erfindungsgemäßen Dichtungsrings ist vorgesehen, dass der Innenring um den Außenring spritzgegossen wird. Sofern der Außenring mindestens einen Durchbruch aufweist, bildet sich auf diese Weise vorteilhafterweise der mindestens eine Steg des Innenrings, welche durch den mindestens einen Durchbruch des Außenrings greift, und der Außenring und der Innenring zueinander formschlüssig angeordnet werden.

In einigen Ausführungsformen des erfindungsgemäßen Dichtungsrings ist vorgesehen, dass der Innenring mindestens eine Nut ausbildet, wobei die Nut einen Boden und zwei gegenüberliegende Seiten aufweist, die sich vom Boden erstrecken, und der Außenring in die Nut formschlüssig eingreift.

In einigen Ausführungsformen des erfindungsgemäßen Dichtungsrings ist vorgesehen, dass der Außenring mindestens einen Durchbruch aufweist, insbesondere ein Langloch, und der Innenring mindestens einen Steg aufweist, der sich von der einen Seite der Nut zur anderen Seite der Nut erstreckt, und der mindestens eine Steg den mindestens einen Durchbruch durchgreift.

In einigen Ausführungsformen des erfindungsgemäßen Dichtungsrings ist vorgesehen, dass der Außenring zwei oder mehrere Durchbrüche bzw. Langlöcher aufweist, insbesondere 3 Durchbrüche oder Langlöcher, und der Innenring entsprechend zwei oder mehrere Stege aufweist, insbesondere 3 Stege aufweist, wobei jeder der zwei oder mehreren Stege jeweils einen der zwei oder mehreren Durchbrüche durchgreift.

In einigen Ausführungsformen des erfindungsgemäßen Dichtungsrings ist vorgesehen, dass das Flüssig-Silikon ausgewählt wird aus einem Zweikomponentengemisch in beliebiger Shore-Härte, welches biokompatibel ist. Gemäß einem Ausführungsbeispiels sind die Komponenten aus der Gruppe SILASTIC® BioMedical Grade Liquid Silicone Rubbers von Dow Corning zu wählen.

Gemäß Anspruch 7 wird eine Kontaktbuchse für einen Verbindungskopf eines implantierbares Geräts zur Verfügung gestellt. Die Kontaktbuchse umfasst dabei einen erfindungsgemäßen Dichtungsring gemäß Anspruch 1 oder einer der vorher beschrieben Ausführungsformen des erfindungsgemäßen Dichtungsrings.

Als Verbindungskopf oder Header im Sinne der vorliegenden Erfindung wird insbesondere eine Baugruppe eines implantierbaren Geräts bezeichnet, die eine elektrische Verbindung zwischen einer stromerzeugenden bzw. stromgebenden Komponente (Generatorkomponente, Batterie) oder einer stromerfassenden Komponente (Diagnosekomponente) eines implantierbaren Geräts und einer Elektrodenleitung gewährleistet.

Insbesondere umfasst die erfindungsgemäße Kontaktbuchse eine Vielzahl von elektrischen Kontakten, wobei jeweils zwischen zwei elektrischen Kontakten ein erfindungsgemäßer Dichtungsring angeordnet ist. Dabei bilden die Vielzahl der elektrischen Kontakte und die dazwischen angeordneten Dichtungsringe einen Hohlraum, insbesondere einen zylindrischen Hohlraum, der zur Aufnahme eines Steckers einer Elektrodenleitung ausgebildet ist. Weiterhin sind die elektrischen Kontakte dazu ausgebildet, elektrische Ströme bzw. Impulse auf den Stecker der Elektrodenleitung zu übertragen oder davon zu empfangen. Typischerweise stehen die elektrischen Kontakte dafür in Kontakt mit einer stromerzeugenden bzw. stromgebenden Komponente (Generatorkomponente, Batterie) oder einer stromerfassenden Komponente (Diagnosekomponente).

Vorzugsweise handelt es sich bei den elektrischen Kontakten der erfindungsgemäßen Kontaktbuchse um Federkontakte. Die Kontaktbuchse kann am Ende oder am Anfang des Elektrodeneingangs auch eine Steckeraufhahme (Connector block) mit Gewindestift aufweisen. Die angrenzende Dichtfunktion durch die Paarung der Komponenten ist vergleichbar mit der hier dargestellten Paarung Dichtring mit elektrischem Kontakt.

Weiterhin wird vorzugsweise die erfindungsgemäße Kontaktbuchse in einen Block aus einem duroplastischen Kunststoff gegossen, wobei dabei ein Verbindungskopf gebildet wird. Vorzugsweise handelt es sich bei dem duroplastischen Kunststoff um einen transparenten Kunststoff, insbesondere ein Epoxidharz.

Gemäß Anspruch 8 wird ein implantierbares Gerät zur Verfügung gestellt, welchen mindestens einen Dichtungsring gemäß Anspruch 1 oder einer der dazugehörigen Ausführungsformen oder eine Kontaktbuchse gemäß Anspruch 7 oder einer der dazugehörigen Ausführungsformen umfasst.

Bei dem implantierbaren Gerät handelt es sich insbesondere um einen Herzschrittmacher, einen Kardioverter-Defibrillator oder einen Neurostimulator wie etwa einen Rückenmarksstimulator.

Bei einem implantierbaren Kardioverter-Defibirillator (ICD) handelt es sich insbesondere um eine Vorrichtung, die einen Stimulationsteil (Impulsgenerator) und einem Diagnostikteil (zur Erkennung bedrohlicher Rhythmusstörungen) aufweist, wobei der Stimulations-/Diagnostikteil, meist unter der Haut in der Nähe des linken Brustmuskels implantiert, über eine Elektrodenleitung mit der rechten Herzkammer verbunden wird. Die Elektrodenleitung wird hierbei typischerweise über die obere Hohlvene in die rechte Herzkammer geführt.

Bei dem Rückenmarksstimulator handelt es sich insbesondere um eine Vorrichtung zur Behandlung von chronischen neuropathischen Schmerzen, in der ein Impulsgenerator über eine Elektrodenleitung mit dem zu behandelnden Teil des Nervensystems verbunden ist, beispielsweise mit dem Hinterstrang des Rückenmarks.

Typischerweise weist das implantierbare Gerät ein Gehäuse aus einem beständigen, biokompatiblen Material auf, vorzugsweise Titan oder eine Titanlegierung, wobei im Gehäuse insbesondere Gerätekomponenten angeordnet sind, die zur bestimmungsgemäßen Verwendung des Gerätes erforderlich sind, wie beispielsweise Steuereinheiten, Energiequellen, Impulsgeneratoren oder Diagnoseeinheiten.

Weitere Einzelheiten und Vorteile der Erfindung sollen durch die nachfolgende Figurenbeschreibung von Ausführungsbeispielen anhand der Figur erläutert werden.

Es zeigen:
- Fig. 1: schematische Darstellungen einer Ausführungsform des erfindungsgemäßen Dichtungsringes;
- Fig. 2: eine schematische Darstellung einer Ausführungsform des erfindungsgemäßen Herstellungsverfahrens; und
- Fig. 3: schematische Darstellungen einer Ausführungsform der erfindungsgemäßen Kontaktbuchse.

### Beispiele:

Die vorliegende Erfindung betrifft insbesondere ein Dichtelement 100 aus einer PSU Komponente 10 und einer Umspritzung 20 aus LSR (*liquid silicone rubber,* Flüssig-Silikon). Hierbei ist die PSU Komponente insbesondere als ein Außenring 10 ausgebildet, und die LSR Umspritzung als ein Innenring 20.

Der PSU-Kern 10 erfüllt dabei folgende Funktionen und weist folgende Vorteile auf:
- Distanz zwischen 2 elektrischen Kontakten
- Anschlag für den Montageprozess
- Anbindungsfläche für Harz zur Bildung des Headers
- mechanische Fixierung zum Silikon (Durchbrüche)
- Anbindungsfläche für Silikon ggf. für Plasmaprozesse oder Primern

Die LSR-Umspritzung 20 erfüllt dabei folgende Funktionen und weist folgende Vorteile auf:
- Potentialtrennung zwischen 2 elektrischen Kontakten (gegeben durch stirnseitigen balligen Dichtflächen des Innenrings, die beim Spannen der Kontaktbuchse zu einer formschlüssigen Verbindung zwischen den elektrischen Kontakten führt)
- Potentialtrennung zwischen Elektrode und Kavität
- Abdichtung gegen Eindringen von Harz beim Harzverguss (realisiert durch die definierte Anschlagsfläche und daraus resultierenden technischen Nullspalt zwischen dem PSU Rind und dem Gehäuse des elektrischen Kontakts)
   Es handelt sich dabei um drei unabhängige Dichtfunktionen.

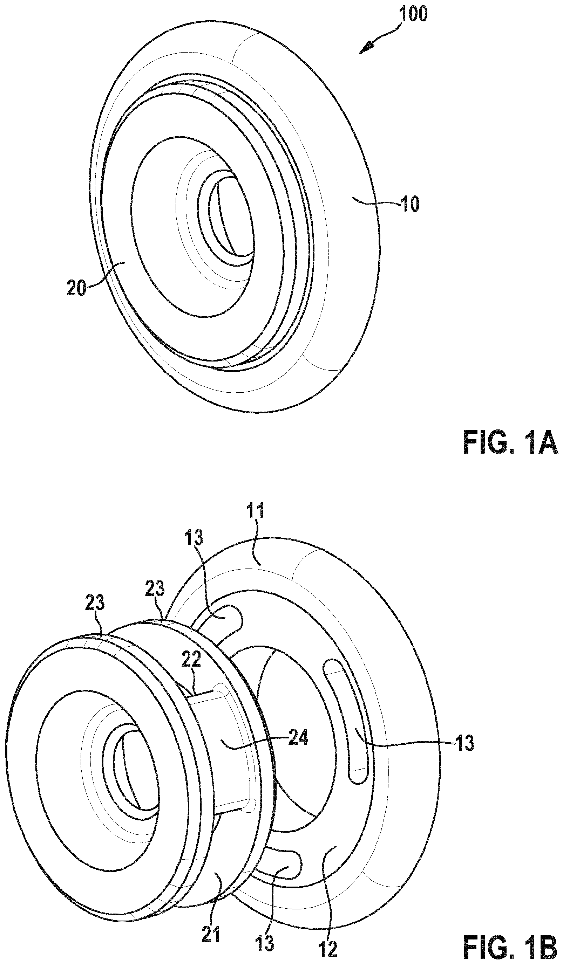

Figur 1A illustriert eine Ausführungsform des erfindungsgemäßen Dichtungsrings 100, welcher aus einem Außenring 10 aus PSU und einem Innenring 20 aus einem Flüssig-Silikon bzw. Flüssigsilikonkautschuk besteht. Figur 1B zeigt jeweils eine Detaildarstellung des Außenrings 10 und des Innenrings 20. Der Außenring 10 weist Langlöcher 13 (Durchbrüche) auf, die zur Verkrallung des Innenrings 20 aus LSR dienen. Der Innenring 20 wiederum bildet eine Nut 21 aus, welche einen Boden 22 und zwei gegenüberliegende Wände umfasst, die sich vom Boden erstrecken. Vorteilhafterweise weist der Außenring 2 oder 3 Stege auf, die sich von einer Wand 23 der Nut 21 zur anderen Wand 23 der Nut 21 erstrecken. Diese Stege 24 durchgreifen die jeweils dazugehörigen Langlöcher des Außenrings 10 und gewährleisten somit eine formschlüssige Verbindung zwischen Innenring 20 und Außenring 10.

Der Außenring 10 dient insbesondere als Anschlag zur Montage der elektrischen Kontakte 30. Aufgrund des verwendeten PSU ist eine besonders gute Anbindung zum Epoxidharz möglich, welche im Zuge der Herstellung des Headers verwendet wird, und somit zur elektrischen Isolierung oberhalb des PSU-Kerns beiträgt. Zusätzlich dient der Außenring als Führung der Verdrahtungsbänder zwischen den elektrischen Kontakten 30, beispielsweise eines 8-pol Moduls, zur Durchführung zum Inneren des Gehäuses. Dies unterstützt den Schweißprozess der Verdrahtungsbänder auf den elektrischen Kontakt 30. Per Design ist eine Potentialtrennung der Verdrahtungsbänder damit ebenso umgesetzt.

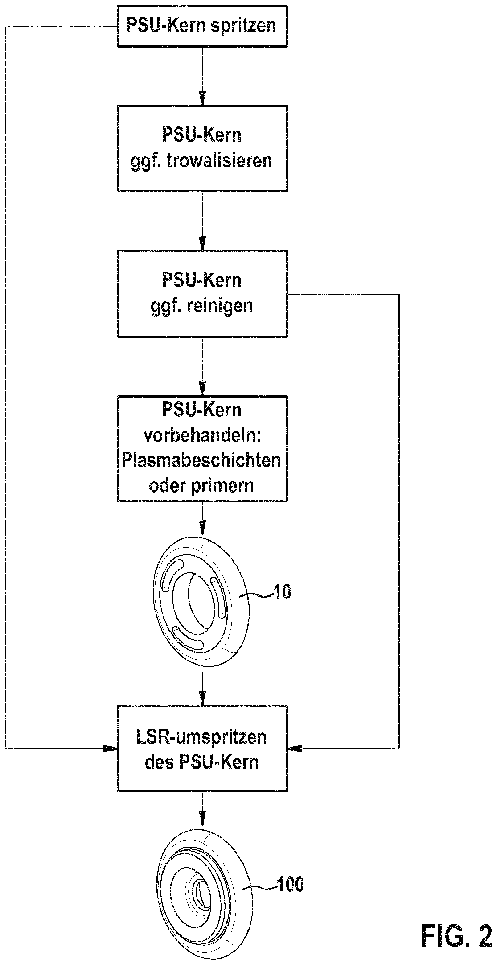

Fig. 2 stellt ein Fließschema der Herstellung des erfindungsgemäßen Dichtungsringes 100 dar. Zunächst wird der PSU-Kern bzw. Außenring 10 durch ein Spritzverfahren bereitgestellt. Ggf. wird der Außenring 10 durch Gleitschleifen (Trowalisieren) oder durch eine Reinigung vorbehandelt. Vorzugsweise wird der Außenring zur besseren Haftung des LSR 20 zusätzlich plasmabearbeitet oder geprimert (Behandlung mit einem Haftvermittler). Anschließend wird LSR um den Außenring 10 gespritzt, wobei das LSR dann den Innenring 20 bildet. Sofern der Außenring 10 Langlöcher 13 aufweist, werden diese vom LSR ausgefüllt, wobei auf diese Weise ein Innenring 20 mit Nut 21 und Stegen 24 entsteht, wobei die Stege 24 dann die jeweiligen Langlöcher 13 durchgreifen, und eine formschlüssige Verbindung zwischen Außenring 10 und Innenring 20 entsteht.

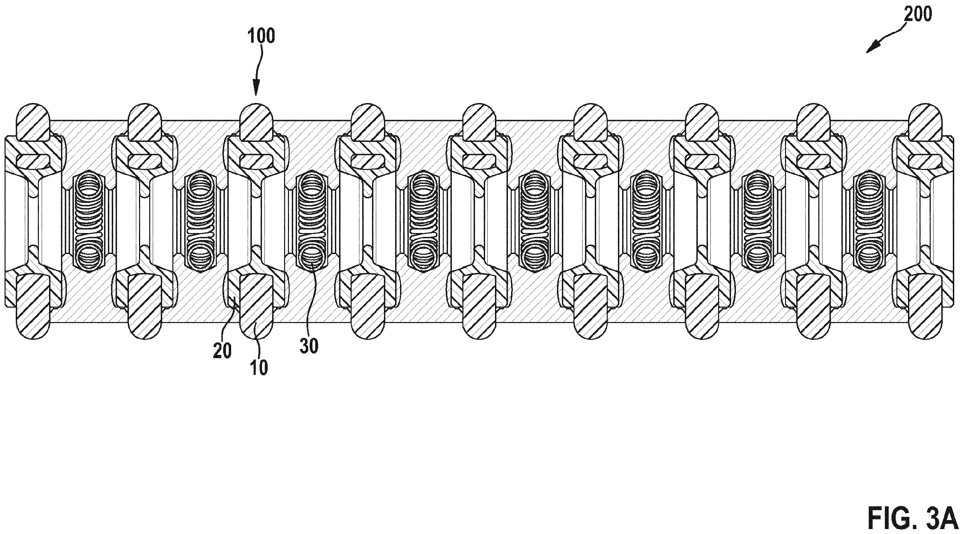

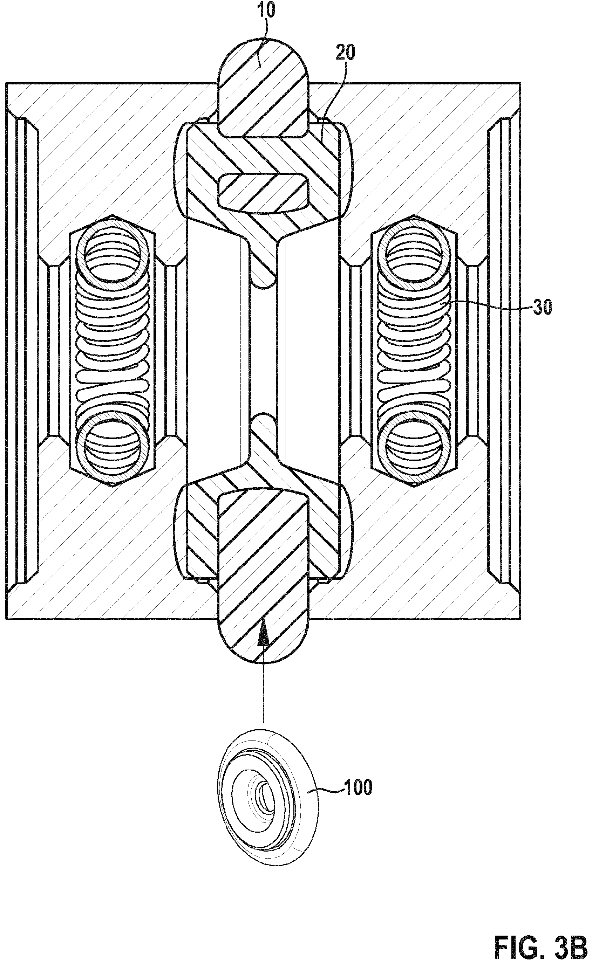

Fig. 3 zeigt schematische Ansichten einer erfindungsgemäßen Kontaktbuchse 200. Die Kontaktbuchse weist eine Vielzahl von elektrischen Kontakten 30 auf, insbesondere Federkontakte, zwischen denen jeweils ein erfindungsgemäßer Dichtungsring 10 angeordnet ist. Die Kontaktbuchse 200 weist im Inneren einen Hohlraum auf, insbesondere einen zylindrischen Hohlraum, der zur Aufnahme eines Steckers einer Elektrodenleitung ausgebildet ist. Hierbei dienen die elektrische Kontakte 30 zur Leitung eines Stromes oder elektrischen Impulses zwischen dem Stecker der Elektrodenleitung und den stromgebenden oder stromerfassenden Komponenten eines implantierbaren Geräts (beispielsweise ICD), die im Gehäuse des Geräts untergebracht sind. Die elektrische Kontakte 30 sind insbesondere über Verdrahtungsbändern mit dem Inneren des Gehäuses verbunden.

## Patentansprüche

1. Dichtungsring (100) für einen Header eines implantierbaren Geräts, aufweisend
- einen Außenring (10), welcher Polysulfon umfasst oder im Wesentlichen daraus besteht, und
- einen Innenring (20), welches ein Flüssig-Silikon umfasst oder im Wesentlichen daraus besteht,
wobei der Innenring (20) und der Außenring (10) zueinander formschlüssig angeordnet sind.

2. Dichtungsring (100) nach Anspruch 1, **dadurch gekennzeichnet, dass** der Innenring (20) mindestens eine Nut (21) ausbildet, wobei die Nut (21) einen Boden (22) und zwei gegenüberliegende Seiten (23) aufweist, die sich vom Boden (22) erstrecken, und der Außenring (10) in die Nut (22) formschlüssig eingreift.

3. Dichtungsring (100) nach Anspruch 2, **dadurch gekennzeichnet, dass** der Außenring (10) mindestens einen Durchbruch (13) aufweist, insbesondere ein Langloch, und der Innenring (20) mindestens einen Steg (24) aufweist, der sich von der einen Seite (23) der Nut (21) zur anderen Seite (23) der Nut (21) erstreckt, und der mindestens eine Steg (24) den mindestens einen Durchbruch (13) durchgreift.

4. Dichtungsring (100) nach einem der vorherigen Ansprüche, **dadurch gekennzeichnet, dass** das Flüssig-Silikon ausgewählt wird aus einem Zweikomponentengemisch in beliebiger Shore-Härte, welches biokompatibel ist.

5. Dichtungsring (100) nach einem der vorherigen Ansprüche, **dadurch gekennzeichnet, dass** der Außenring (10) spritzgegossen wird.

6. Dichtungsring (100) nach einem der vorherigen Ansprüche, **dadurch gekennzeichnet, dass** der Innenring (20) um den Außenring (10) spritzgegossen wird.

7. Kontaktbuchse (200) für ein implantierbares Gerät umfassend mindestens einen Dichtungsring (100) nach einem der Ansprüche 1 bis 6.

8. Implantierbares Gerät umfassend einen Dichtungsring (100) nach einem Ansprüche 1 bis 6 oder eine Kontaktbuchse (200) nach Anspruch 7, insbesondere ein implantierbarer Kardioverter-Defibrillator oder ein implantierbarer Rückenmarksstimulator.

9. Verfahren zur Herstellung eines Dichtungsrings (100) nach einem der Ansprüche 1 bis 5, umfassend die Schritte:
- Bereitstellen eines Außenrings (10), welcher Polysulfon umfasst oder im Wesentlichen daraus besteht,
- Bereitstellen eines Innenrings (20), welches ein Flüssig-Silikon umfasst oder im Wesentlichen daraus besteht, und
- Formschlüssiges Anordnen des Außenrings (10) und des Innenrings (20) zueinander.

10. Verfahren nach Anspruch 9, **dadurch gekennzeichnet, dass** der Innenring (20) mindestens eine Nut (21) ausbildet, wobei die Nut (21) einen Boden (22) und zwei gegenüberliegende Seiten (23) aufweist, die sich vom Boden (22) erstrecken, und der Außenring (10) in die Nut (21) formschlüssig eingreift.

11. Verfahren nach Anspruch 10, **dadurch gekennzeichnet, dass** der Außenring (10) mindestens einen Durchbruch (13) aufweist, insbesondere ein Langloch, und der Innenring (20) mindestens einen Steg (24) aufweist, der sich von der eine Seite (23) der Nut (21) zur anderen Seite (23) der Nut (21) erstreckt, und der mindestens eine Steg (24) den mindestens einen Durchbruch (13) durchgreift.

12. Verfahren nach einem der vorherigen Ansprüche 9 bis 11, **dadurch gekennzeichnet, dass** der Außenring (10) durch ein Spritzverfahren bereitgestellt wird, wobei insbesondere der Außenring (10) nach dem Spritzen geschliffen oder gereinigt wird.

13. Verfahren nach einem der vorherigen Ansprüche 9 bis 12, **dadurch gekennzeichnet, dass** der Innenring (20) durch ein Spritzverfahren bereitgestellt wird.

14. Verfahren nach einem der vorherigen Ansprüche 9 bis 13, **dadurch gekennzeichnet, dass** der Innenring (20) um den Außenring (10) gespritzt wird und dabei formschlüssig zu dem Außenring (10) angeordnet wird.

15. Verfahren nach einem der vorherigen Ansprüche 9 bis 14, **dadurch gekennzeichnet, dass** Außenring (10) vor dem formschlüssigen Anordnen, insbesondere vor dem Umspritzen des Innenrings (20), vorbehandelt wird, insbesondere unter Verwendung eines Plasma oder eines Haftvermittlers (Primern).
